# EUROPÄISCHE PATENTSCHRIFT

(11) **EP 3 376 261 B1**
(45) Veröffentlichungstag und Bekanntmachung des Hinweises auf die Patenterteilung: **29.04.2020**
(21) Anmeldenummer: 17161126.2
(22) Anmeldetag: 15.03.2017
(51) Int. Cl.: G01T 1/24, G01T 1/29

(54) **RÖNTGENDETEKTOR AUFWEISEND EIN KONVERTERELEMENT MIT UMVERDRAHTUNGSEINHEIT**
X-RAY DETECTOR COMPRISING A CONVERTER ELEMENT WITH REWIRING UNIT
DÉTECTEUR DE RAYONS X PRÉSENTANT UN ÉLÉMENT DE CONVERTISSEUR COMPRENANT UNE UNITÉ DE RECÂBLAGE

(43) Veröffentlichungstag der Anmeldung: 19.09.2018
(73) Patentinhaber: Siemens Healthcare GmbH, 91052 Erlangen (DE)
(72) Erfinder: ERGLER, Thorsten, 91054 Erlangen (DE); GEYER, Harald, 91088 Bubenreuth (DE); SCHROETER, Christian, 96050 Bamberg (DE); TONN, Justus, 91301 Forchheim (DE); WREGE, Jan, 91056 Erlangen (DE)

(56) Entgegenhaltungen:
- WO-A1-2015/012866
- WO-A1-2016/046014
- US-A1- 2012 133 001
- US-A1- 2012 223 241

## Beschreibung

Die Erfindung betrifft einen Röntgendetektor aufweisend ein Konverterelement mit einer an einer der Auswerteeinheit zugewandten Oberfläche des Konverterelements ausgebildeten Umverdrahtungseinheit, sowie ein medizinisches Gerät und ein Herstellungsverfahren dazu.

In der Röntgenbildgebung, beispielsweise in der Computertomographie, der Angiographie oder der Radiographie, können zählende direkt-konvertierende Röntgendetektoren oder integrierende indirekt-konvertierende Röntgendetektoren verwendet werden.

Die Röntgenstrahlung oder die Photonen können in direktkonvertierenden Röntgendetektoren durch ein geeignetes Konvertermaterial in elektrische Pulse umgewandelt werden. Als Konvertermaterial können beispielsweise CdTe, CZT, CdZnTeSe, CdTeSe, CdMnTe, InP, TlBr2, HgI2, GaAs oder andere verwendet werden. Die elektrischen Pulse, beispielsweise Spannungspulse, werden von einer Auswerteelektronik, beispielsweise einem integrierten Schaltkreis (Application Specific Integrated Circuit, ASIC), in einer Auswerteeinheit bewertet. In zählenden Röntgendetektoren wird einfallende Röntgenstrahlung durch Zählen der elektrischen Pulse, welche durch die Absorption von Röntgenphotonen im Konvertermaterial ausgelöst werden, gemessen. Die Höhe des elektrischen Pulses ist in der Regel proportional zur Energie des absorbierten Röntgenphotons. Dadurch kann eine spektrale Information durch den Vergleich der Höhe des elektrischen Pulses mit einem Schwellwert extrahiert werden. Die Auswerteeinheit kann mit einer Trägereinheit, beispielsweise einer Trägerkeramik, verbunden sein, so dass eine erhöhte mechanische Stabilität erreicht werden kann und eine Umverdrahtung der Signalverbindungen hin zu nachfolgenden Signalverarbeitungseinheiten bereitgestellt werden kann. Um dem Einfall gestreuter Röntgenphotonen entgegenzuwirken, kann ein Streustrahlengitter auf der der Strahlungsquelle zugewandte Seite des Konverterelements ausgebildet sein.

Aus der DE 10 2012 213 410 B3 ist ein direkt-konvertierender Röntgenstrahlungsdetektor bekannt, welcher mindestens eine auf einem Halbleiter aufgebrachte Elektrode aufweist. Die mindestens eine Elektrode und der Halbleiter sind elektrisch leitend verbunden, wobei die mindestens eine Elektrode transparent und elektrisch leitend ausgebildet ist.

Aus der DE 101 38 913 A1 ist ein Detektormodul für Röntgen-Computertomographen bekannt, wobei auf einer Vorderseite einer Leiterplatte ein eine Vielzahl von Sensorelementen aufweisendes Sensorarray montiert ist. Zur Erhöhung der Genauigkeit des Detektors ist erfindungsgemäß vorgesehen, dass an der dem Sensorarray abgewandten Rückseite der Leiterplatte mindestens ein Heizelement zum Beheizen des Sensorarrays und einer in der Nähe des Heizelements angeordneten Regelelektronik zum Regeln des Heizelements vorgesehen ist.

Aus der US 2012/223241 A1 ist ein direktkonvertierenden Röntgendetektor bekannt, welcher eine Umverteilungslage mit einem Substrat und einer isolierenden Schicht auf einer Unterseite des Substrats aufweist, wobei als erstes Substrat ein Silizium-Substrat offenbart wird.

Aus der WO 2015/012866 A1 ist ein Röntgendetektor mit einer Detektionsuntereinheit und deiner Elektronikuntereinheit bekannt, wobei die Detektionsuntereinheit ein Isolatorelement aufweist, in welchem elektrische Leitungen ausgebildet sind, welche es erleichtern können, Teile der Detektionsuntereinheit von der Elektronikuntereinheit abzuschirmen.

Aus der US 2012/133001 A1 ist Detektor mit einer Zwischenlage bekannt, welche eine Umverteilungslage umfasst. Dabei kann die Zwischenlage über einen Interposer mit der Sensormatrix verbunden sein, welcher als flexibler oder als ein nichtflexibler Interposer ausgebildet sein kann.

Aus der WO 2016/046014 A1 ist ein Strahlungsdetektor mit einem Interposer bekannt, welcher ein Heizelement aufweist.

Der Erfindung liegt das Problem zugrunde, dass die Auswerteeinheit und das Konverterelement eine im Wesentlichen gleiche flächige Erstreckung aufweisen, um einen mechanisch stabilen Stapelaufbau zu erhalten, und damit die Kosten hoch sind, da die Kosten der Auswerteeinheit von der flächigen Erstreckung der Auswerteeinheit abhängen.

Es ist Aufgabe der Erfindung, einen Röntgendetektor, ein medizinisches Gerät und ein Verfahren zur Herstellung eines Röntgendetektors anzugeben, welche einen kostengünstigen und mechanisch stabilen Röntgendetektor ermöglichen.

Die Aufgabe wird erfindungsgemäß gelöst durch einen Röntgendetektor nach Anspruch 1, ein medizinisches Gerät nach Anspruch 9 und ein Verfahren nach Anspruch 11.

Die Erfindung betrifft einen zählenden Röntgendetektor aufweisend eine Stapelanordnung mit einem Konverterelement, einer Umverdrahtungseinheit und einer Auswerteeinheit. Das Konverterelement weist eine erste Elektrode an einer der Auswerteeinheit abgewandten Oberfläche und eine pixelierte zweite Elektrode mit einer Mehrzahl von Elektrodenelementen an einer der Auswerteeinheit zugewandten Oberfläche auf. Die Auswerteeinheit weist Pixelelektroden auf. Die Umverdrahtungseinheit ist an einer der Auswerteeinheit zugewandten Oberfläche des Konverterelements ausgebildet, wobei erste Kontakte von zwischen den Elektrodenelementen und den Pixelelektroden elektrisch leitenden Verbindungen an einer dem Konverterelement zugewandten Oberfläche der Umverdrahtungseinheit vorgesehen sind und eine erste flächige Verteilung aufweisen, und zweite Kontakte von den zwischen den Elektrodenelementen und den Pixelelektroden elektrisch leitenden Verbindungen an einer der Auswerteeinheit zugewandten Oberfläche der Umverdrahtungseinheit vorgesehen sind und eine im Vergleich zur ersten flächigen Verteilung kleinere zweite flächige Verteilung aufweisen. Eine flächige Erstreckung der Auswerteeinheit ist kleiner als eine flächige Erstreckung des Konverterelements.

Das, insbesondere direkt-konvertierende, flächenhafte Konverterelement und die flächenhafte Auswerteeinheit sind in einer Stapelanordnung übereinander angeordnet, wobei die flächenhaften Ausdehnungen parallel zueinander verlaufen. Das Konverterelement und die Auswerteeinheit können über elektrisch leitende Verbindungen in der Umverdrahtungseinheit und Lotverbindungen bzw. Klebeverbindungen elektrisch leitend verbunden sein, insbesondere können die Elektrodenelemente und die Pixelelektroden elektrisch leitend verbunden sein. Die elektrisch leitenden Verbindungen zwischen den Elektrodenelementen und den Pixelelektroden können im Wesentlichen gleich lang oder unterschiedlich lang sein. Die Lotverbindungen bzw. die Klebeverbindungen können insbesondere zwischen der Umverdrahtungseinheit und der Auswerteeinheit ausgebildet sein. Die Lotverbindungen bzw. die Klebeverbindungen können zwischen den zweiten Kontakten und den Pixelelektroden ausgebildet sein.

Die Umverdrahtungseinheit kann mittels eines lithographischen Verfahrens direkt auf der der Auswerteeinheit zugewandten Oberfläche des Konverterelements hergestellt werden. Die Umverdrahtungseinheit kann direkt auf dem Konverterelement ausgebildet sein. Die Umverdrahtungseinheit kann untrennbar mit dem Konverterelement verbunden sein. Der Anschlussbereich wird von den Elektrodenelementen hin zu den Pixelelektroden verkleinert. Die flächige Erstreckung der Auswerteeinheit kann beispielsweise um einen Faktor 4 kleiner sein als eine flächige Erstreckung des Konverterelements. Der Faktor kann beispielsweise Werte im Bereich von 1,5 bis 5 annehmen.

Die erste Elektrode kann als flächenhafte oder strukturierte Elektrode ausgebildet sein. Die pixelierte zweite Elektrode weist eine Mehrzahl von Elektrodenelementen auf. Die Anzahl der Elektrodenelemente bestimmt die Anzahl der Pixel bzw. Detektorelemente. Zwischen der ersten Elektrode und der pixelierten zweiten Elektrode mit einer Mehrzahl von Elektrodenelementen kann eine Hochspannung angelegt sein, so dass die durch die einfallende Röntgenstrahlung ausgelösten Elektronen-Loch-Paare getrennt werden und elektrische Pulse in der Auswerteeinheit registriert werden. Die Hochspannung kann beispielsweise -1000V betragen. Die Umverdrahtungseinheit und die mit der Umverdrahtungseinheit verbundene Auswerteeinheit können insbesondere mittig auf dem Konverterelement angeordnet sein.

Die der Auswerteeinheit zugewandten Oberfläche des Konverterelements befindet sich im Betrieb auf der der Strahlungsquelle abgewandten Oberfläche des Konverterelements. Die der Auswerteeinheit abgewandte Oberfläche des Konverterelements befindet sich im Betrieb auf der der Strahlungsquelle zugewandten Oberfläche des Konverterelements.

Jedem Elektrodenelement kann eine Pixelelektrode zugeordnet sein. Jedes Elektrodenelement kann insbesondere mit einer Pixelelektrode mittels elektrisch leitender Verbindungen der Umverdrahtungseinheit elektrisch leitend verbunden sein. Die ersten Kontakte der elektrisch leitenden Verbindungen sind in elektrisch leitender Verbindung mit den Elektrodenelementen. Die zweiten Kontakte der elektrisch leitenden Verbindungen sind in elektrisch leitender Verbindung mit Pixelelektroden.

Die erste flächige Verteilung oder/und die zweite flächige Verteilung können auch als Dichte der ersten Kontakte bzw. zweiten Kontakte bezeichnet werden. Insbesondere können die Abstände zwischen benachbarten ersten Kontakten größer sein als die Abstände zwischen benachbarten zweiten Kontakten. Die erste flächige Verteilung der ersten Kontakte kann der flächigen Verteilung der Elektrodenelemente im Wesentlichen entsprechen. Die zweite flächige Verteilung der zweiten Kontakte kann der flächigen Verteilung der Pixelelektroden im Wesentlichen entsprechen. Die ersten Kontakte können, insbesondere jeweils, im Wesentlichen gleiche flächige Erstreckungen wie die Elektrodenelemente aufweisen. Die zweiten Kontakte können, insbesondere jeweils, im Wesentlichen gleiche flächige Erstreckungen wie die Pixelelektroden aufweisen. Jede Pixelelektrode kann eine kleinere flächige Erstreckung aufweisen im Vergleich zu einem Elektrodenelement. Die Pixelelektrode kann beispielsweise um einen Faktor 4 kleiner sein im Vergleich zu einem Elektrodenelement. Der Faktor kann beispielsweise im Bereich von 1,5 bis 5 liegen.

Vorteilhaft können die Kosten zur Herstellung reduziert werden. Vorteilhaft kann auf einen sogenannten Interposer zur Umverdrahtung verzichtet werden. Vorteilhaft kann die Fläche der Auswerteeinheit reduziert sein. Die Umverdrahtungseinheit direkt auf dem Konverterelement kann einen sogenannten Interposer zur Umverdrahtung vorteilhaft kostengünstig ersetzen. Die Umverdrahtungseinheit ist vorteilhaft mechanisch stabil, insbesondere auch unter Temperaturveränderungen im Betrieb. Vorteilhaft kann die Integrationsdichte des Röntgendetektors erhöht werden. Vorteilhaft können kleinere Subeinheiten des Röntgendetektors realisiert werden. Vorteilhaft kann die Aufbaukomplexität des Röntgendetektors reduziert werden. Durch die Entkopplung der Pixelstrukturen im Konverterelement und der Auswerteeinheit kann vorteilhaft eine höhere Flexibilität bei der relativen Positionierung der Auswerteeinheit in Bezug zum Konverterelement ermöglicht werden. Vorteilhaft können die Spaltmaße zwischen benachbarten Auswerteinheiten bzw. Auswerteeinheit und Konverterelement relativ frei gewählt werden.

Gemäß einem Aspekt der Erfindung umfassen die elektrisch leitenden Verbindungen der Umverdrahtungseinheit zumindest einen ersten Abschnitt senkrecht zu einer Flächennormale der der Auswerteeinheit zugewandten Oberfläche des Konverterelements und zumindest einen zweiten Abschnitt parallel zur Flächennormale der der Auswerteeinheit zugewandten Oberfläche des Konverterelements. Mittels zumindest des ersten Abschnitts und des zweiten Abschnitts der elektrisch leitenden Verbindung kann die erste flächige Verteilung auf eine kleinere zweite flächige Verteilung reduziert werden. Vorteilhaft kann können die Elektrodenelemente mit den Pixelelektroden der im Vergleich zum Konverterelement kleineren Auswerteeinheit verbunden werden. Vorteilhaft umfasst die Umverdrahtungseinheit ein elektrisch isolierendes Material.

Erfindungsgemäß weist die Umverdrahtungseinheit zumindest eine elektrisch isolierende Schicht auf. Die elektrisch isolierende Schicht kann die elektrisch leitenden Verbindungen gegenüber dem Konverterelement, der Auswerteeinheit und benachbarten weiteren elektrisch leitenden Verbindungen elektrisch isolieren. Vorteilhaft sind die elektrischen Signale nicht gestört.

Gemäß einem Aspekt der Erfindung ist der erste Abschnitt senkrecht zur Flächennormalen der der Auswerteeinheit zugewandten Oberfläche des Konverterelements zumindest teilweise von der zumindest einen elektrisch isolierenden Schicht umgeben oder/und der zweite Abschnitt parallel zur Flächennormalen der der Auswerteeinheit zugewandten Oberfläche des Konverterelements zumindest teilweise von der zumindest einen elektrisch isolierenden Schicht oder/und einer weiteren elektrisch isolierenden Schicht umgeben. Besonders vorteilhaft sind der erste Abschnitt oder/und der zweite Abschnitt vollständig von der isolierenden Schicht oder/und einer weiteren isolierenden Schicht umgeben. Vorteilhaft kann innerhalb der isolierenden Schicht bzw. der isolierenden Schichten eine Umverdrahtung mittels der elektrisch leitenden Verbindungen ausgebildet sein.

Erfindungsgemäß weist die Umverdrahtungseinheit eine metallische Zwischenschicht auf. Die elektrischen Feldlinien im Konverterelement sind vorteilhaft unbeeinflusst von der Umverdrahtungseinheit bzw. den elektrisch leitenden Verbindungen der Umverdrahtungseinheit. Der Leitungswiderstand kann vorteilhaft sehr gering sein. Die etwaige metallische Zwischenschicht kann derart als Abschirmung dienen, dass die elektrischen Feldlinien im Konverterelement bzw. die elektrischen Signale durch die Umverdrahtungseinheit im Wesentlichen unbeeinflusst sind. Die Abschirmung kann durch mehrere metallische Zwischenschichten in der Umverdrahtungseinheit ausgebildet sein.

Gemäß einem Aspekt der Erfindung weist die Umverdrahtungseinheit eine flexible Zwischenschicht auf. Die etwaige flexible Zwischenschicht kann mechanische Verspannungen im Röntgendetektor minimieren, so dass Signalinstabilitäten vorteilhaft reduziert bzw. vermieden werden. Die flexible Zwischenschicht kann als Teilschritt in einem lithographischen Prozess hergestellt werden. Die flexible Zwischenschicht kann ein geeignetes, flexibles oder weiches, insbesondere elektrisch isolierendes, Material aufweisen. Das flexible Material kann mechanische Verspannungen ausgleichen.

Gemäß einem Aspekt der Erfindung weist der Röntgendetektor ferner ein Heizelement zur Einstellung einer Temperatur des Konverterelements auf. Die Temperatur des Röntgendetektors, insbesondere des Konverterelements, kann mittels des etwaigen Heizelements vorteilhaft stabilisiert bzw. geregelt werden. Das Heizelement kann beispielsweise an der Trägereinheit angeordnet sein. Das Heizelement kann beispielsweise an der ersten Elektrode angeordnet sein. Das Heizelement kann beispielsweise eine Peltier-Element oder eine elektrisch betreibbare Heizschleife sein.

Gemäß einem Aspekt der Erfindung ist die erste Elektrode für sichtbares Licht, infrarotes Licht oder ultraviolettes Licht transparent ausgebildet. Die erste Elektrode kann transparent ausgebildet sein, um eine zusätzliche Beleuchtung des Konverterelements mit infrarotem, ultraviolettem oder sichtbarem Licht zu ermöglichen.

Gemäß einem Aspekt der Erfindung weist der Röntgendetektor ferner eine Beleuchtungseinheit zur Beleuchtung des Konverterelements mit sichtbarem Licht, infrarotem Licht oder ultraviolettem Licht auf. Mittels der zusätzlichen Beleuchtung kann ein Polarisationszustand des Konverterelements bestimmt, festgelegt oder korrigiert werden.

Gemäß einem Aspekt der Erfindung ist die erste Elektrode für sichtbares Licht, infrarotes Licht oder ultraviolettes Licht intransparent ausgebildet. Die erste Elektrode kann intransparent, insbesondere für infrarotes, ultraviolettes oder sichtbares Licht, sein.

Gemäß einem Aspekt der Erfindung weist der Röntgendetektor ferner eine mit der Auswerteeinheit verbundene Trägereinheit auf. Die Trägereinheit kann den Röntgendetektor vorteilhaft mechanisch stabilisieren. Die Trägereinheit kann mit der Auswerteeinheit mechanisch und insbesondere auch elektrisch leitend verbunden sein, beispielsweise mittels einer Lotverbindung oder einer Klebeverbindung, so dass die Signale der Auswerteeinheit über die Trägereinheit vom Röntgendetektor weggeführt werden können.

Gemäß einem Aspekt der Erfindung ist die Trägereinheit zumindest mechanisch mit der Umverdrahtungseinheit verbunden. Die Trägereinheit kann mit der Konvertereinheit zumindest mechanisch verbunden sein, beispielsweise mittels einer Lotverbindung oder einer Klebeverbindung. Die Trägereinheit kann den Röntgendetektor vorteilhaft mechanisch stabilisieren.

Die Erfindung betrifft ferner ein medizinisches Gerät aufweisend einen erfindungsgemäßen Röntgendetektor. Vorteilhaft können die Vorteile des erfindungsgemäßen Röntgendetektors auf das erfindungsgemäße medizinische Gerät übertragen werden. Vorteilhaft können die Kosten zur Herstellung gesenkt werden.

Gemäß einem Aspekt der Erfindung ist das medizinische Gerät ein Computertomographiesystem. Vorteilhaft kann die mechanische Stabilität den Anforderungen eines rotierenden Röntgendetektors genügen.

Die Erfindung betrifft ferner ein Verfahren zur Herstellung eines Röntgendetektors aufweisend die Schritte des Bereitstellens, des Aufbringens und des Verbindens. Im Schritt des Bereitstellens wird eine Konverterelements aufweisend eine erste Elektrode an einer ersten Oberfläche und eine pixelierte zweite Elektrode mit einer Mehrzahl von Elektrodenelementen an einer zweiten Oberfläche und eine Auswerteeinheit mit Pixelelektroden bereitgestellt. Im Schritt des Aufbringens wird eine Umverdrahtungseinheit an der zweiten Oberfläche des Konverterelements aufgebracht, wobei erste Kontakte von zwischen den Elektrodenelementen und den Pixelelektroden elektrisch leitenden Verbindungen vorgesehen sind und eine erste flächige Verteilung an einer dem Konverterelement zugewandten Oberfläche der Umverdrahtungseinheit aufweisen und zweite Kontakte von den zwischen den Elektrodenelementen und den Pixelelektroden elektrisch leitenden Verbindungen an einer der Auswerteeinheit zugewandten Oberfläche der Umverdrahtungseinheit vorgesehen sind und eine im Vergleich zur ersten Verteilung kleinere zweite flächige Verteilung aufweisen. Im Schritt des Verbindens wird die Umverdrahtungseinheit mit der Auswerteeinheit mittels elektrisch leitender Verbindungen zwischen den zweiten Kontakten und den Pixelelektroden verbunden.

Das Verbinden kann die Verwendung eines elektrisch leitenden Klebers oder einer Lotverbindung umfassen. Der etwaige Lötprozess zum Verbinden des Konverterelements mittels Lotverbindung mit der Auswerteeinheit findet vorteilhaft auf der Umverdrahtungseinheit, aufweisend eine isolierende Schicht, statt. Vorteilhaft kann die Eindiffusion von Lotmaterial in das Konverterelement vermieden werden. Vorteilhaft kann auf eine sogenannte Under-Bump-Metallisierung verzichtet werden bzw. diese vereinfacht werden. Vorteilhaft kann die Umverdrahtungseinheit Verspannungen durch das etwaige Einbringen einer Unterfüllung reduzieren. Vorteilhaft können Signalinstabilitäten, sogenannte Drift-Spots, vermieden werden.

Gemäß einem Aspekt der Erfindung umfasst der Schritt des Aufbringens ein lithographisches Verfahren. Vorteilhaft können die elektrisch leitenden Verbindungen und die isolierende Schicht in einem lithographischen Verfahren aufgebraucht werden.

Erfindungsgemäß umfasst der Schritt des Aufbringens das Aufbringen mindestens einer isolierenden Schicht. Das Aufbringen kann wie folgt durchgeführt werden: Auf das bereitgestellte Konverterelement wird, beispielsweise mittels einer Maske, eine isolierende Schicht aufgebracht, wobei bevorzugt die Elektrodenelemente freigelassen werden. Auf die freigelassenen Elektrodenelemente kann ein elektrisch leitendes Material aufgebracht werden, so dass ein erste Kontakt entsteht. Auf die isolierende Schicht kann eine erste Verbindung aufweisend ein elektrisch leitendes Material aufgebracht werden. Anschließend kann mittels einer weiteren Maske eine weitere isolierende Schicht aufgebracht werden, wobei zumindest eine Stelle zum Ankontaktieren der zweiten Verbindung freigelassen wird. Es wird mittels eines elektrisch leitenden Materials ab der freigelassenen Stelle die zweite Verbindung ausgebildet. Es wird mittels eines elektrischen Materials ab der zweiten Verbindung die dritte Verbindung ausgebildet, deren Ende der zweite Kontakt ist. Es kann optional eine zusätzliche isolierende Schicht aufgebracht werden, welche die nach dem Verbinden die Auswerteeinheit zumindest teilweise berührt.

Erfindungsgemäß umfasst der Schritt des Aufbringens das Aufbringen einer metallischen Zwischenschicht.Gemäß einem Aspekt der Erfindung umfasst der Schritt des Aufbringens das Aufbringen einer metallischen Zwischenschicht und einer flexiblen Zwischenschicht. Die metallische Zwischenschicht oder/und die flexible Zwischenschicht können insbesondere auf eine isolierende Zwischenschicht aufgebracht werden. Die metallische Zwischenschicht kann insbesondere von der elektrisch leitenden Verbindung elektrisch isoliert sein. Die elektrisch leitenden Verbindungen können durch die metallische Zwischenschicht bzw. die flexible Zwischenschicht hindurch verlaufen, so dass eine durchgehende elektrisch leitende Verbindung ausgebildet ist.

Nachfolgend werden Ausführungsbeispiele der Erfindung anhand von Zeichnungen näher erläutert. Hierbei zeigt:
FIG 1 schematisch einen Röntgendetektor in einer Ausführungsform;
FIG 2 schematisch einen erfindungsgemäßen Röntgendetektor in einer Ausführungsform;
FIG 3 schematisch einen Röntgendetektor in einer Ausführungsform;
FIG 4 schematisch einen erfindungsgemäßen Röntgendetektor in einer Ausführungsform;
FIG 5 schematisch eine Umverdrahtungseinheit des erfindungsgemäßen Röntgendetektors in einer Draufsicht;
FIG 6 schematisch eine Umverdrahtungseinheit des erfindungsgemäßen Röntgendetektors in einer Seitenansicht;
FIG 7 schematisch eine Darstellung eines erfindungsgemäßen Computertomographiesystems; und
FIG 8 schematisch eine Darstellung des erfindungsgemäßen Verfahrens.

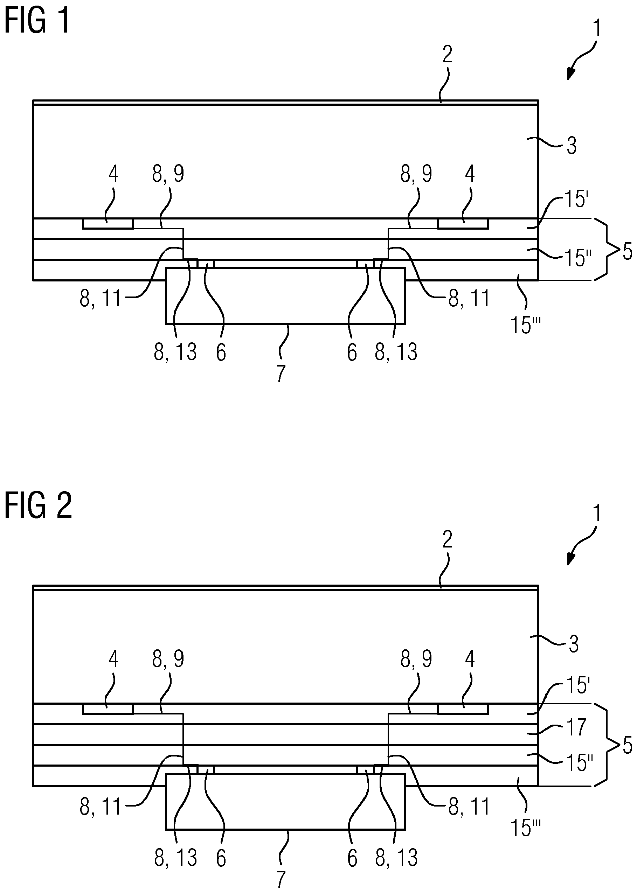

Die Fig. 1 zeigt eine beispielhafte Ausführung des erfindungsgemäßen Röntgendetektors 1 in einer ersten Ausführungsform. Der Röntgendetektor 1 weist eine Stapelanordnung mit einem Konverterelement 3, einer Umverdrahtungseinheit 5 und einer Auswerteeinheit 7 auf. Das Konverterelement 3 weist eine erste Elektrode 2 an einer der Auswerteeinheit 7 abgewandten Oberfläche und eine pixelierte zweite Elektrode mit einer Mehrzahl von Elektrodenelementen 4 an einer der Auswerteeinheit 7 zugewandten Oberfläche auf. Die Auswerteeinheit 7 weist Pixelelektroden 6 auf. Die Umverdrahtungseinheit 5 ist an einer der Auswerteeinheit 7 zugewandten Oberfläche des Konverterelements 3 ausgebildet, wobei erste Kontakte von zwischen den Elektrodenelementen 4 und den Pixelelektroden 6 elektrisch leitenden Verbindungen 8, 9, 11, 13 an einer dem Konverterelement 3 zugewandten Oberfläche der Umverdrahtungseinheit 5 vorgesehen sind und eine erste flächige Verteilung aufweisen, und zweite Kontakte von den zwischen den Elektrodenelementen 4 und den Pixelelektroden 6 elektrisch leitenden Verbindungen 8, 9, 11, 13 an einer der Auswerteeinheit 7 zugewandten Oberfläche der Umverdrahtungseinheit 5 vorgesehen sind und eine im Vergleich zur ersten flächigen Verteilung kleinere zweite flächige Verteilung aufweisen. Eine flächige Erstreckung der Auswerteeinheit 7 ist kleiner als eine flächige Erstreckung des Konverterelements 3.

Die elektrisch leitenden Verbindungen 8, 9, 11, 13 der Umverdrahtungseinheit 5 umfassen zumindest einen ersten Abschnitt 9 senkrecht zu einer Flächennormalen der der Auswerteeinheit 7 zugewandten Oberfläche des Konverterelements 3 und zumindest einen zweiten Abschnitt 11 parallel zur Flächennormalen der der Auswerteeinheit 7 zugewandten Oberfläche des Konverterelements 3 umfassen. Die Umverdrahtungseinheit 5 weist eine elektrisch isolierende Schichten 15', 15", 15'" auf.

Der erste Abschnitt 9 senkrecht zur Flächennormalen der der Auswerteeinheit 7 zugewandten Oberfläche des Konverterelements 3 ist zumindest teilweise von der zumindest einen elektrisch isolierenden Schicht 15' umgeben oder/und der zweite Abschnitt 11 parallel zur Flächennormalen der der Auswerteeinheit 7 zugewandten Oberfläche des Konverterelements 3 ist zumindest teilweise von der zumindest einen elektrisch isolierenden Schicht 15' und weiteren elektrisch isolierenden Schichten 15", 15'" umgeben.

Die Fig. 2 zeigt den erfindungsgemäßen Röntgendetektors 1. Die Umverdrahtungseinheit 5 weist eine metallische Zwischenschicht 17 auf.

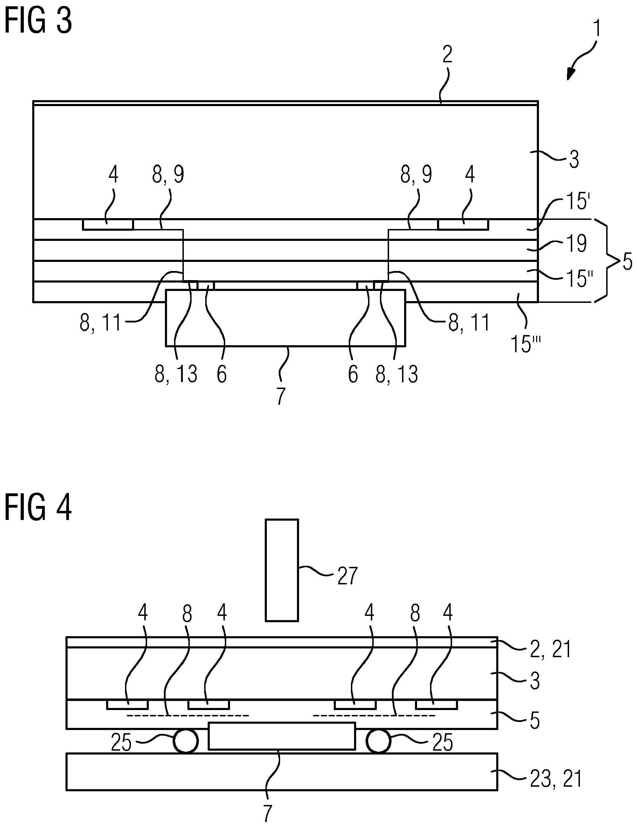

Die Fig. 3 zeigt eine beispielhafte Ausführung des erfindungsgemäßen Röntgendetektors 1 in einer weiteren Ausführungsform. Die Umverdrahtungseinheit 5 weist eine flexible Zwischenschicht 19 auf.

Die Fig. 4 zeigt eine beispielhafte Ausführung des erfindungsgemäßen Röntgendetektors 1 in einer weiteren Ausführungsform. Der Röntgendetektor 1 weist ferner eine mit der Auswerteeinheit 7 verbundene Trägereinheit 23 auf. Der Röntgendetektor 1 weist ferner ein Heizelement 21 zur Einstellung einer Temperatur des Konverterelements 3 auf. Das Heizelement 21 ist von der Trägereinheit 23 oder/und von der ersten Elektrode 2 umfasst. Die Trägereinheit 23 ist zumindest mechanisch mittels Lotverbindungen 25 mit der Umverdrahtungseinheit 5 verbunden. Der Röntgendetektor 1 weist ferner ein Streustrahlengitter 27 auf. Schematisch ist nur eine Gitterwand des Streustrahlengitters 27 gezeigt.

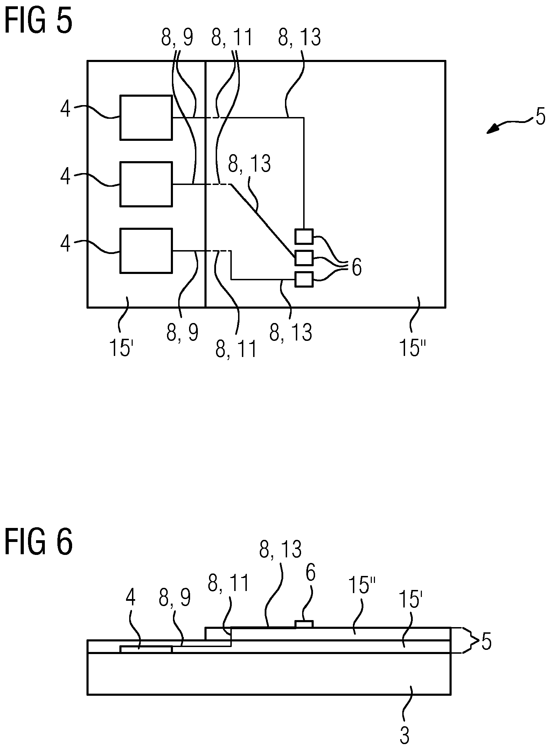

Die Fig. 5 zeigt eine beispielhafte Ausführung einer Umverdrahtungseinheit 5 des erfindungsgemäßen Röntgendetektors 1 in einer Draufsicht. Die flächige Erstreckung der isolierenden Schicht 15' ist größer als die flächige Erstreckung der isolierenden Schicht 15". Die erste Verbindung 8,9 verläuft auf der Oberfläche der isolierenden Schicht 15'. Die zweite Verbindung 8, 11 verbindet die erste Verbindung 8, 9 mit der dritten Verbindung 8, 13. Die dritte Verbindung 8, 13 verläuft auf der Oberfläche der isolierenden Schicht 15". Die Pixelelektroden 6 weisen eine kleinere flächige Erstreckung als die Elektrodenelemente 4 auf.

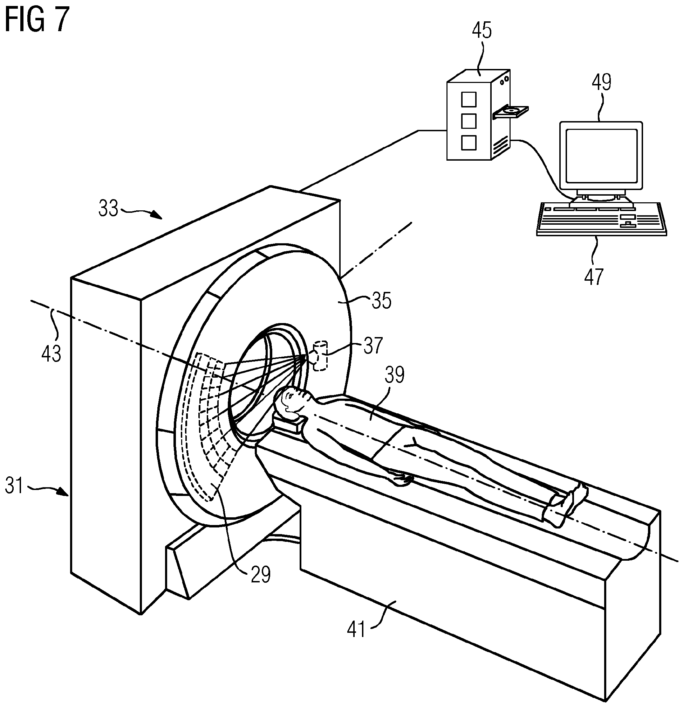

Die Fig. 6 zeigt eine beispielshafte Ausführung einer Umverdrahtungseinheit 5 direkt auf dem Konverterelement 3 des erfindungsgemäßen Röntgendetektors 1 in einer Seitenansicht. Die Fig. 7 zeigt eine beispielhafte Ausführung eines erfindungsgemäßen Computertomographiesystems 31 mit einem erfindungsgemäßen Röntgendetektor. Das Computertomographiesystem 31 beinhaltet eine Gantry 33 mit einem Rotor 35. Der Rotor 35 umfasst eine Röntgenquelle 37 und einer Detektorvorrichtung 29 aufweisend mindestens einen erfindungsgemäßen Röntgendetektor. Der Patient 39 ist auf der Patientenliege 41 gelagert und ist entlang der Rotationsachse z 43 durch die Gantry 33 bewegbar. Zur Steuerung und Berechnung der Schnittbilder wird eine Recheneinheit 45 verwendet. Eine Eingabeeinrichtung 47 und eine Ausgabevorrichtung 49 sind mit der Recheneinheit 45 verbunden.

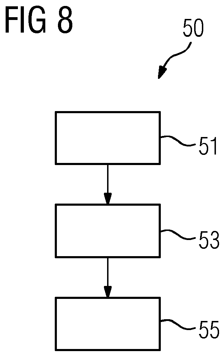

Die Fig. 8 zeigt eine beispielhafte Darstellung des erfindungsgemäßen Verfahrens 50 zur Herstellung eines Röntgendetektors aufweisend die Schritte des Bereitstellens 51, des Aufbringens 53 und des Verbindens 55. Im Schritt des Bereitstellens 51 wird ein Konverterelement aufweisend eine erste Elektrode an einer ersten Oberfläche und eine pixelierte zweite Elektrode mit einer Mehrzahl von Elektrodenelementen an einer zweiten Oberfläche bereitgestellt und eine Auswerteeinheit mit Pixelelektroden bereitgestellt. Im Schritt des Aufbringens 53 wird eine Umverdrahtungseinheit an der zweiten Oberfläche des Konverterelements aufgebracht, wobei erste Kontakte von zwischen den Elektrodenelementen und den Pixelelektroden elektrisch leitenden Verbindungen vorgesehen sind und eine erste flächige Verteilung an einer dem Konverterelement zugewandten Oberfläche der Umverdrahtungseinheit aufweisen und zweite Kontakte von den zwischen den Elektrodenelementen und den Pixelelektroden elektrisch leitenden Verbindungen an einer der Auswerteeinheit zugewandten Oberfläche der Umverdrahtungseinheit vorgesehen sind und eine im Vergleich zur ersten Verteilung kleinere zweite flächige Verteilung aufweisen. Der Schritt des Aufbringens 53 umfasst das Aufbringen einer isolierenden Schicht 15' auf das Konverterelement 3 und das Aufbringen einer metallischen Zwischenschicht 17. Der Schritt des Aufbringens 53 umfasst ein lithographisches Verfahren. Im Schritt des Verbindens 55 wird die Umverdrahtungseinheit mit der Auswerteeinheit mittels elektrisch leitender Verbindungen zwischen den zweiten Kontakten und den Pixelelektroden verbunden.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung, welcher durch die beigefügten Schutzansprüche bestimmt wird, zu verlassen.

## Patentansprüche

1. Zählender Röntgendetektor (1) aufweisend eine Stapelanordnung mit einem Konverterelement (3), einer Umverdrahtungseinheit (5) und einer Auswerteeinheit (7), wobei
a. das Konverterelement (3) eine erste Elektrode (2) an einer der Auswerteeinheit (7) abgewandten Oberfläche und eine pixelierte zweite Elektrode mit einer Mehrzahl von Elektrodenelementen (4) an einer der Auswerteeinheit (7) zugewandten Oberfläche aufweist,
b. die Auswerteeinheit (7) Pixelelektroden (6) aufweist,
c. die Umverdrahtungseinheit (5) an einer der Auswerteeinheit (7) zugewandten Oberfläche des Konverterelements (3) ausgebildet ist, wobei
d. die Umverdrahtungseinheit (5) zumindest eine isolierende Schicht (15'), welche auf dem Konverterelement (3) aufgebracht ist, aufweist, und
e. erste Kontakte von zwischen den Elektrodenelementen (4) und den Pixelelektroden (6) elektrisch leitenden Verbindungen (8, 9, 11, 13) an einer dem Konverterelement (3) zugewandten Oberfläche der Umverdrahtungseinheit (5) vorgesehen sind und eine erste flächige Verteilung aufweisen, und
f. zweite Kontakte von den zwischen den Elektrodenelementen (4) und den Pixelelektroden (6) elektrisch leitenden Verbindungen (8, 9, 11, 13) an einer der Auswerteeinheit (7) zugewandten Oberfläche der Umverdrahtungseinheit (5) vorgesehen sind und eine im Vergleich zur ersten flächigen Verteilung kleinere zweite flächige Verteilung aufweisen, und
g. eine flächige Erstreckung der Auswerteeinheit (7) kleiner ist als eine flächige Erstreckung des Konverterelements (3), **dadurch gekennzeichnet dass**
die Umverdrahtungseinheit (5) eine metallische Zwischenschicht (17) aufweist.

2. Röntgendetektor (1) nach Anspruch 1, wobei die elektrisch leitenden Verbindungen (8, 9, 11, 13) der Umverdrahtungseinheit (5) zumindest einen ersten Abschnitt (9) senkrecht zu einer Flächennormalen der der Auswerteeinheit (7) zugewandten Oberfläche des Konverterelements (3) und zumindest einen zweiten Abschnitt (11) parallel zur Flächennormalen der der Auswerteeinheit (7) zugewandten Oberfläche des Konverterelements (3) umfassen.

3. Röntgendetektor (1) nach einem der vorangehenden Ansprüche, wobei die Umverdrahtungseinheit (5) zumindest eine weitere elektrisch isolierende Schicht (15", 15'") aufweist.

4. Röntgendetektor (1) nach den Ansprüchen 2 und 3, wobei der erste Abschnitt (9) senkrecht zur Flächennormalen der der Auswerteeinheit (7) zugewandten Oberfläche des Konverterelements (3) zumindest teilweise von der zumindest einen elektrisch isolierenden Schicht (15') umgeben ist oder/und der zweite Abschnitt (11) parallel zur Flächennormalen der der Auswerteeinheit (7) zugewandten Oberfläche des Konverterelements (3) zumindest teilweise von der zumindest einen elektrisch isolierenden Schicht (15') oder/und einer weiteren elektrisch isolierenden Schicht (15", 15'") umgeben ist.

5. Röntgendetektor (1) nach einem der vorangehenden Ansprüche, wobei die Umverdrahtungseinheit (5) eine flexible Zwischenschicht (19) aufweist.

6. Röntgendetektor (1) nach einem der vorangehenden Ansprüche, ferner aufweisend ein Heizelement (21) zur Einstellung einer Temperatur des Konverterelements (3).

7. Röntgendetektor (1) nach einem der vorangehenden Ansprüche, ferner aufweisend eine mit der Auswerteeinheit (7) verbundene Trägereinheit (23).

8. Röntgendetektor (1) nach Anspruch 7, wobei die Trägereinheit (23) zumindest mechanisch mit der Umverdrahtungseinheit (5) verbunden ist.

9. Medizinisches Gerät aufweisend einen Röntgendetektor (1) nach einem der Ansprüche 1 bis 8.

10. Medizinisches Gerät nach Anspruch 9, wobei das medizinische Gerät ein Computertomographiesystem (31) ist.

11. Verfahren (50) zur Herstellung eines Röntgendetektors (1) aufweisend die Schritte:
a. Bereitstellen (51) eines Konverterelements (3) aufweisend eine erste Elektrode (2) an einer ersten Oberfläche und eine pixelierte zweite Elektrode mit einer Mehrzahl von Elektrodenelementen (4) an einer zweiten Oberfläche und Bereitstellen (51) einer Auswerteeinheit (7) mit Pixelelektroden (6),
b. Aufbringen (53) einer Umverdrahtungseinheit (5) an der zweiten Oberfläche des Konverterelements (3), wobei der Schritt des Aufbringens (53) das Aufbringen einer isolierenden Schicht (15') auf das Konverterelement (3) und das Aufbringen einer metallischen Zwischenschicht (17) umfasst, und wobei erste Kontakte von zwischen den Elektrodenelementen (4) und den Pixelelektroden (6) elektrisch leitenden Verbindungen (8, 9, 11, 13) vorgesehen sind und eine erste flächige Verteilung an einer dem Konverterelement (3) zugewandten Oberfläche der Umverdrahtungseinheit (5) aufweisen und zweite Kontakte von den zwischen den Elektrodenelementen (4) und den Pixelelektroden (6) elektrisch leitenden Verbindungen (8, 9, 11, 13) an einer der Auswerteeinheit (7) zugewandten Oberfläche der Umverdrahtungseinheit (5) vorgesehen sind und eine im Vergleich zur ersten Verteilung kleinere zweite flächige Verteilung aufweisen, und
c. Verbinden (55) der Umverdrahtungseinheit (5) mit der Auswerteeinheit (7) mittels elektrisch leitender Verbindungen zwischen den zweiten Kontakten und den Pixelelektroden (6).

12. Verfahren (50) nach Anspruch 11, wobei der Schritt des Aufbringens (53) ein lithographisches Verfahren umfasst.

13. Verfahren (50) nach Anspruch 11 oder 12, wobei der Schritt des Aufbringens (53) das Aufbringen mindestens einer weiteren isolierenden Schicht (15", 15'") umfasst.

14. Verfahren (50) nach einem der Ansprüche 11 bis 13, wobei der Schritt des Aufbringens (53) das Aufbringen einer metallischen Zwischenschicht (17) und einer flexiblen Zwischenschicht (19) umfasst.

## Claims

1. Counting x-ray detector (1) having a stack arrangement comprising a converter element (3), a rewiring unit (5) and an evaluation unit (7), wherein
a. the converter element (3) has a first electrode (2) on a surface facing away from the evaluation unit (7) and a pixelated second electrode having a plurality of electrode elements (4) on a surface facing toward the evaluation unit (7),
b. the evaluation unit (7) has pixel electrodes (6),
c. the rewiring unit (5) is embodied on a surface of the converter element (3) facing toward the evaluation unit (7), wherein
d. the rewiring unit (5) has at least one insulating layer (15') which is deposited onto the converter element (3),
e. first contacts of electrically conductive connections (8, 9, 11, 13) between the electrode elements (4) and the pixel electrodes (6) are provided on a surface of the rewiring unit (5) facing toward the converter element (3) and have a first areal distribution, and
f. second contacts of the electrically conductive connections (8, 9, 11, 13) between the electrode elements (4) and the pixel electrodes (6) are provided on a surface of the rewiring unit (5) facing toward the evaluation unit (7) and have a smaller second areal distribution compared to the first areal distribution, and
g. an areal extent of the evaluation unit (7) is smaller than an areal extent of the converter element (3), **characterised in that** the rewiring unit (5) has a metallic intermediate layer (17).

2. X-ray detector (1) according to claim 1, wherein the electrically conductive connections (8, 9, 11, 13) of the rewiring unit (5) comprise at least one first section (9) perpendicular to a surface normal of the surface of the converter element (3) facing toward the evaluation unit (7) and at least one second section (11) parallel to the surface normal of the surface of the converter element (3) facing toward the evaluation unit (7).

3. X-ray detector (1) according to one of the preceding claims, wherein the rewiring unit (5) has at least one further electrically insulating layer (15", 15'").

4. X-ray detector (1) according to claims 2 and 3, wherein the first section (9) perpendicular to the surface normal of the surface of the converter element (3) facing toward the evaluation unit (7) is at least partially surrounded by the at least one electrically insulating layer (15') or/and the second section (11) parallel to the surface normal of the surface of the converter element (3) facing toward the evaluation unit (7) is at least partially surrounded by the at least one electrically insulating layer (15') or/and a further electrically insulating layer (15", 15'").

5. X-ray detector (1) according to one of the preceding claims, wherein the rewiring unit (5) has a flexible intermediate layer (19).

6. X-ray detector (1) according to one of the preceding claims, additionally having a heating element (21) for setting a temperature of the converter element (3).

7. X-ray detector (1) according to one of the preceding claims, additionally having a carrier unit (23) connected to the evaluation unit (7).

8. X-ray detector (1) according to claim 7, wherein the carrier unit (23) is connected at least mechanically to the rewiring unit (5).

9. Medical device having an x-ray detector (1) according to one of claims 1 to 8.

10. Medical device according to claim 9, wherein the medical device is a computed tomography system (31).

11. Method (50) for producing an x-ray detector (1), comprising the steps:
a. providing (51) a converter element (3) having a first electrode (2) on a first surface and a pixelated second electrode having a plurality of electrode elements (4) on a second surface, and providing (51) an evaluation unit (7) having pixel electrodes (6),
b. depositing (53) a rewiring unit (5) on the second surface of the converter element (3), wherein the step of depositing (53) comprises the depositing of an insulating layer (15') onto the converter element (3) and the depositing of a metallic intermediate layer (17), and wherein first contacts of electrically conductive connections (8, 9, 11, 13) between the electrode elements (4) and the pixel electrodes (6) are provided and have a first areal distribution on a surface of the rewiring unit (5) facing toward the converter element (3), and second contacts of the electrically conductive connections (8, 9, 11, 13) between the electrode elements (4) and the pixel electrodes (6) are provided on a surface of the rewiring unit (5) facing toward the evaluation unit (7) and have a smaller second areal distribution compared to the first distribution, and
c. connecting (55) the rewiring unit (5) to the evaluation unit (7) by means of electrically conductive connections between the second contacts and the pixel electrodes (6).

12. Method (50) according to claim 11, wherein the depositing step (53) comprises a lithographic process.

13. Method (50) according to claim 11 or 12, wherein the depositing step (53) comprises the deposition of at least one further insulating layer (15'', 15'").

14. Method (50) according to one of claims 11 to 13, wherein the depositing step (53) comprises the deposition of a metallic intermediate layer (17) and a flexible intermediate layer (19).

## Revendications

1. Détecteur (1) de rayons X compteur, comportant un agencement d'empilement ayant un élément (3) de convertisseur, une unité (5) de recâblage et une unité (7) d'exploitation, dans lequel
a. l'élément (3) de convertisseur a une première électrode (2) sur une surface loin de l'unité (7) d'exploitation et une deuxième électrode pixelée ayant une pluralité d'éléments (4) d'électrode sur une surface tournée vers l'unité (7) d'exploitation,
b. l'unité (7) d'exploitation a des électrodes (6) de pixel,
c. l'unité (5) de recâblage est constituée sur une surface, tournée vers l'unité (7) d'exploitation, de l'élément (3) de convertisseur, dans lequel
d. l'unité (5) de recâblage a au moins une couche (15') isolante, déposée sur l'élément (3) de convertisseur,
e. des premiers contacts de liaison (8, 9, 11, 13) conductrices de l'électricité, entre les éléments (4) d'électrode et les électrodes (6) de pixel, sont prévus sur une surface, tournée vers l'élément (3) de convertisseur, de l'unité (5) de recâblage et ont une première répartition en surface, et
f. des deuxièmes contacts de liaison (8, 9, 11, 13) conductrices de l'électricité, entre les éléments (4) d'électrode et les électrodes (6) de pixel, sont prévus sur une surface, tournée vers l'unité (7) d'exploitation de l'unité (5) de recâblage, et ont une deuxième répartition en surface plus petite que la première répartition en surface, et
g. une étendue en surface de l'unité (7) d'exploitation est plus petite qu'une étendue en surface de l'élément (3) de convertisseur, **caractérisé en ce que** l'unité (5) de recâblage a une couche (17) intermédiaire métallique.

2. Détecteur (1) de rayons X suivant la revendication 1, dans lequel les liaisons (8, 9, 11, 13) conductrices de l'électricité de l'unité (5) de recâblage comprennent au moins une première partie (9) perpendiculaire à une normale à la surface, tournée vers l'unité (7) d'exploitation, de l'élément (3) de convertisseur, et au moins une deuxième partie (11) parallèle à la normale à la surface, tournée vers l'unité (7) d'exploitation, de l'élément (3) de convertisseur.

3. Détecteur (1) de rayons X suivant l'une des revendications précédentes, dans lequel l'unité (5) de recâblage a au moins une autre couche (15", 15'") isolante du point de vue électrique.

4. Détecteur (1) de rayons X suivant les revendications 2 et 3, dans lequel la première partie (9), perpendiculaire à la normale à la surface, tournée vers l'unité (7) d'exploitation, de l'élément (3) de convertisseur, est entourée, au moins en partie, de la au moins une couche (15') isolante du point de vue électrique ou/et la deuxième (11), parallèle à la normale à la surface, tournée vers l'unité (7) d'exploitation, de l'élément (3) de convertisseur, est entourée, au moins en partie, de la au moins une couche (15') isolante du point de vue électrique ou/et d'une autre couche (15", 15'") isolante du point de vue électrique.

5. Détecteur (1) de rayons X suivant l'une des revendications précédentes, dans lequel l'unité (5) de recâblage a une couche (19) intermédiaire souple.

6. Détecteur (1) de rayons X suivant l'une des revendications précédentes, comportant, en outre, un élément (21) de chauffage pour régler une température de l'élément (3) de convertisseur.

7. Détecteur (1) de rayons X suivant l'une des revendications précédentes, comportant, en outre, une unité (23) de support reliée à l'unité (7) d'exploitation.

8. Détecteur (1) de rayons X suivant la revendication 7, dans lequel l'unité (23) de support est reliée au moins mécaniquement à l'unité (5) de recâblage.

9. Appareil médical ayant un détecteur (1) de rayons X suivant l'une des revendications 1 à 8.

10. Appareil médical suivant la revendication 9, dans lequel l'appareil médical est un système (31) de tomographie assisté par ordinateur.

11. Procédé (50) de fabrication d'un détecteur (1) à rayons X, comportant les stades :
a. on se procure (51) un élément (3) de convertisseur ayant une première électrode (2) sur une première surface et une deuxième électrode pixelée ayant une pluralité d'éléments (4) d'électrode sur une deuxième surface et on se procure (51) une unité (7) d'exploitation ayant des électrodes (6) de pixel,
b. on dépose (53) une unité (5) de recâblage sur la deuxième surface de l'élément (3) de convertisseur, le stade du dépôt (53) comportant le dépôt d'une couche (15') isolante sur l'élément (3) de convertisseur et le dépôt d'une couche (17) intermédiaire métallique, et dans lequel des premiers contacts de liaisons (8, 9, 11, 13) conductrices de l'électricité, entre les éléments (4) d'électrode et les électrodes (6) de pixel, sont prévus et ont une première répartition en surface sur une surface tournée vers l'élément (3) de convertisseur, et des deuxièmes contacts de liaisons (8, 9, 11, 13) conductrices de l'électricité, entre les éléments (4) d'électrode et les électrodes (6) de pixel, sont prévus sur une surface, tournée vers l'unité (7) d'exploitation, de l'unité (5) de recâblage et ont une deuxième répartition en surface plus petite que la première répartition, et
c. on connecte (55) l'unité (5) de recâblage à l'unité (7) d'exploitation au moyen de liaisons conductrices de l'électricité entre les deuxièmes contacts et les électrodes (6) de pixel.

12. Procédé (50) suivant la revendication 11, dans lequel le stade du dépôt (53) comprend un procédé lithographique.

13. Procédé (50) suivant la revendication 11 ou 12, dans lequel le stade du dépôt (53) comprend le dépôt d'au moins une autre couche (15", 15'") isolante.

14. Procédé (50) suivant l'une des revendications 11 à 13, dans lequel le stade du dépôt (53) comprend le dépôt d'une couche (17) intermédiaire métallique et d'une couche (19) intermédiaire souple.
